# EUROPEAN PATENT SPECIFICATION

(11) **EP 1 163 904 B1**
(45) Date of publication and mention of the grant of the patent: **19.04.2006**
(21) Application number: 00112811.5
(22) Date of filing: 16.06.2000
(51) Int. Cl.: A61K 31/195, A61K 31/375, A61K 33/14, A61P 9/00, A61P 11/00, A61P 27/00

(54) **Composition for the prevention of smooth muscle diseases comprising ascorbate, arginine and magnesium**
Zusammensetzung für die Verhinderung der Glattmuskelkrankheiten, die Ascorbat, Arginin und Magnesium enthält
Composition pour la prévention des maladies de muscle lise comprenant l'ascorbate, l'arginine et le magnésium

(43) Date of publication of application: 19.12.2001
(73) Proprietor: Rath, Matthias, Dr. med., 15th Floor Cape Town, 8001 (ZA)
(72) Inventor: Rath, Matthias, Dr. med., 15th Floor Cape Town, 8001 (ZA)
(74) Representative: Federhen, Ludwig

(56) References cited:
- EP-A- 0 891 771
- WO-A-01/22958
- WO-A-99/51252
- GB-A- 2 029 220
- GB-A- 2 268 871
- US-A- 5 198 465
- US-A- 5 626 883
- US-A- 5 650 418
- US-A- 5 891 459
- RATH M. ET AL: "Nutritional supplement program halts progression of early coronary atherosclerosis documented by ultrafast computed tomography" JOURNAL OF APPLIED NUTRITION, 1996, 48/3 (68-78), XP002050411 USA
- BOSTOM A. G.: "The effect of high dose ascorbate supplementation on plasma lipoprotein (a) levels in patients with premature coronary heart disease" PHARMACOTHERAPY, vol. 15, no. 4, July 1995 (1995-07), pages 458-464, XP000972298

## Description

### Technical field:

The present invention relates to the prevention and treatment of health conditions caused by constriction of smooth muscle cells in organs of the human body, like tinnitus, asthma, glaucoma, infertility, spasms of the ureter and urethra, singultus, stomach cramps as cramps of the gall ducts.

### Background of the invention:

The cause of many diseases remains unknown. Among these diseases with unknown origin are most common diseases including asthma, glaucoma and tinnitus. Worldwide several hundred million people suffer from these health conditions and the economic damage to society from not being able to treat these health conditions effectively is immeasurable.

Worldwide several million people suffer from asthma bronchiale (asthma). In its late stages asthma is a debilitating disease leading to the inability to work and to social isolation. The cause of this disease remains unknown, even though allergens, genetic disposition and psychological factors have been implicated. The common pathomechanism of this disease is an obstruction of the ventilation channels in the lung (bronchioles) and of the passages to the alveoli where the oxygenation takes place. However, the cellular mechanisms that trigger this obstruction, thereby causing asthma, is not yet understood.

Tinnitus is a form of hearing impairment that in most cases occurs suddenly and without any warning signs. It is estimated that worldwide more than one million patients suffer from tinnitus and related hearing impairment. The origin and the pathogenesis of this disease are unknown and currently there exists no effective therapy to improve the hearing capacity of tinnitus patients. Consequently, many of these patients suffer for years and decades from impaired hearing often with detrimental consequences for the ability to work and their social life.

It is assumed that a variety of apparently different health conditions occurring in different organs is caused by biochemical dysfunction of the same type of cells, called smooth muscle cells. This type of muscle cell is not susceptible to conscious control as opposed for example to the muscles of the arms or legs. Proper metabolic function of smooth muscle cells would, therefore, be a key to avoiding health problems in all those organs where smooth muscle cells play a critical functional role.

In case of asthma it is assumed that the common cellular mechanism could be found. The walls of bronchioles and alveoli channels contain a layer of smooth muscle cells. If these cells would constrict ― irrespective of the trigger ― the diameter of these lung ducts would decrease, leading to a decrease in ventilation and oxygenation. Thus, the typical symptoms of a patient with asthma and/or obstructive lung disease would occur.

In case of tinnitus and hearing impairment it is conceivable that the hearing impairment is caused by spasm of smooth muscle cells which form the lining of blood vessels responsible for blood supply to the inner ear and to the nerve cells mediating acoustic signals. It was further assumed that the spasm of these smooth muscle cells in the ear capillaries is caused by a lack of co-enzymes and other bioenergy molecules essential for optimum metabolic function of these cells.

There was not found any earlier description of this concept in the scientific literature.

Recent progress has been made in understanding the metabolism of cells and the role of certain biochemical compounds in maintaining their proper function. Consequently, correcting the dysfunction of smooth muscle cell metabolism would be a key to preventing and treating a variety of health conditions in different organs.

GB 2268871 discloses a food supplement containing a protein or peptide, a vitamin, an amino acid, and magnesium. This food supplement is useful for the enhancement of heart function and circulation, which is not an object of the present invention.

Rath et al.: Journal of Applied Nutrition (1996) 48/3, pages 68 - 78 disclose a nutrient supplement containing arginine, vitamin C, and magnesium for preventing heart diseases and stopping progression of coronary atherosclerosis, i. e. angina pectoris, which is also not an object of the present invention.

WO 99/51252 discloses a dietary supplement comprising, inter alia, arginine, vitamin C, and magnesium. This supplement may be used to facilitate smooth muscle relaxation and vascular dilatation. However, this document does not describe the use of a composition comprising arginine, vitamin C, and magnesium compounds for the treatment of the specific diseases which are addressed by the present invention.

WO 01/22958 discloses a composition comprising arginine, vitamin C, and magnesium for treating diseases associated with atherosclerosis and plaque instability, which is also not an object of the present invention.

It was further found that the cellular dysfunction could be caused by a deficiency of certain biochemical compounds needed as co-enzymes in the tricarbon acid-cycle, the so-called trebs-cycle, the respriation chain and for other metabolic functions in smooth muscle cells.

The health conditions that can potentially be prevented and treated include the following organs and diseases:
Lungs, eye, uro-genital tract, gastro-intestinal tract. Tinnitus, impotence, asthma and other forms of obstructive lung diseases, glaucoma and other forms of increased eye pressure, pre-menstrual syndrome, infertility, spasms of the ureter, urethra, singultus, stomach cramps, spasms of the gall duct

### Summary of the invention:

It is therefore an object of this invention to administer a composition of biochemical substances to a patient suffering from health conditions as mentioned above consisting of at least one ascorbate compound selected from the group consisting of ascorbic acid, pharmaceutically acceptable ascorbate salts and/or mixtures thereof with at least one arginine compound selected from the group of arginine hydrochlorides, pharmaceutically acceptable arginine salts and/or mixtures thereof and at least one magnesium compound selected from magnesium or pharmaceutically acceptable magnesium salts and/or mixtures thereof.

It is a further object of this invention to administer to a patient the therapeutical composition of biochemical substances like

Ascorbic Acid, Ascorbyl Palmitate, Beta-, Gamma-, Delta-Tocopherol-Mix, Beta-Carotene, Biotin, Calcium Ascorbate, Calcium Citrate, Calcium Glycinate, Carotinoid-Mix: _{(Alpha-Carot., Lutein, Zea-, Kryptoxanthin)}, Cholecalciferol, Chromium Glycinate, Citrus Bioflavonoids, Coenzyme Q10, Copper Glycinate, Cyanocobalamin, d-Alpha-Tocopherol, d-Calcium Pantothenate, Dicalcium Phosphate, Folic Acid, Inositol, L-Arginine, L-Camitine, L-Cysteine, L-Lysine, L-Proline, L-Selenomethionine, Magnesium Ascorbate, Magnesium Citrate, Magnesim Glycinate, Manganese, Chelate, Molybdenum Glycinate, Niacin, Niacinamid, Potassium Chelate, Pycnogenol, Pyridoxine, Riboflavin, Thiamine, Zinc Glycinate, irrespective of dosage of these added components.

It is a further object of this invention to administer to a patient a composition of biochemical substances whereas these formulas are provided to a patient in form of tablets, pills, injections, infusions, inhalations, suppositories or other pharmaceutically acceptable carriers and/or means of delivery.

This concept was clinically tested for several of the above-mentioned health conditions, i. e. asthma and tinnitus.

The treatment with such compositions of biochemical substances leads to at least partly considerable relaxations of smooth muscle cells resulting in the increase of diameter of arterioles and capillaries (e.g. arteries of the ear) leading to improved hearing, relaxation of smooth muscle cells in lung bronchioles and alveoli leading to an increase of airway diameter following a decrease of asthma symptoms, the relaxation of canal systems of the eye resulting in an increase of diameter e.g. of tear ducts decreasing eye pressure leading to a decreased risk of glaucoma and blindness, the relaxation of smooth muscle cells in ovarian tubes and uterus resulting in relaxation of muscle tissue improving fertility and decreasing PMS symptoms, relaxation of smooth muscle cells in gall ducts, ureter and urethra increasing the diameter of ducts resulting in a decreased risk of cramps caused by gall stones or kidney stones.

Having disclosed a preferred embodiment of the present invention, the following examples are provided by way of illustration only.

### Examples:

Compositions of biochemical substances as listed below have been administered patients in the daily amounts of units of the substances shown in this example

| Biochemical Substances | Units | Amount |
|---|---|---|
| Ascorbic Acid | mg | 680 |
| Ascorbyl Palmitate | mg | 620 |
| Beta-, Gamma-, Delta-Tocopherol-Mix | mg | 22 |
| Beta-Carotene | I.U. | 1665 |
| Biotin | mcg | 65 |
| Calcium Ascorbate | mg | 1050 |
| Calcium Citrate | mg | 200 |
| Calcium Glycinate | mg | 35 |
| Carotinoid-Mix: _{(Alpha-Carot., Lutein, Zea-, Kryptoxanthin)} | mcg | 50 |
| Cholecalciferol | I.U. | 130 |
| Chromium Glycinate | mcg | 10 |
| Citrus Bioflavonoids | mg | 650 |
| Coenzyme Q10 | mg | 7 |
| Copper Glycinate Cyanocobalamin | mcg | 330 |
| | mcg | 20 |
| d-Alpha-Tocopherol d-Calcium Pantothenate | LU. | 230 |
| | mg | 40 |
| Dicalcium Phosphate | mg | 15 |
| Folic Acid | mcg | 90 |
| Inositol | mg | 35 |
| L-Arginine | mg | 790 |
| L-Carnitine | mg | 35 |
| L-Cysteine L-Lysine | mg | 35 |
| | mg | 110 |
| L-Proline | mg | 110 |
| L-Selenomethionine | mcg | 20 |
| Magnesium Ascorbate | mg | 1050 |
| Magnesium Citrate Magnesium Glycinate | mg | 400 |
| | mg | 40 |
| Manganese Chelate | mcg | 1300 |
| Molybdenum Glycinate Niacin | mcg | 4 |
| | mg | 10 |
| Niacinamid | mg | 35 |
| Potassium Chelate | mg | 20 |
| Pycnogenol | mg | 7 |
| Pyridoxine | mg | 10 |
| Riboflavin | mg | 7 |
| Thiamine | mg | 7 |
| Zinc Glycinate | mg | 7 |

| | | |
|---|---|---|
| mg = milligrams, | | |
| mcg = Micrograms, | | |
| I.U. = International Units | | |

However, a composition of biochemical substances according to this example may be varied with individual components, others than of the formula used and inespective of their amounts which are more than 80% identical with such substances. Further the amounts of individual ingredients provided per day may be not less than 10% and not more than 1000% of the amounts as shown in the example.

The concept of this invention was tested in a prospective clinical study with eight asthma patients. These patients received the biochemical compounds listed above as a daily dosage for a period of four months. At the beginning and at the end of the study the lung volume was measured in each patient. During the study the asthma patients increased their lung volume on average by more than 20%. The most significant result of this study was the fact that the percentage of lung volume increase was much higher for those patients with the lowest base line values which indicates that this therapy is particularly valuable with patients with severe asthma and a severe breathing impairment as shown in graph 1 hereof:

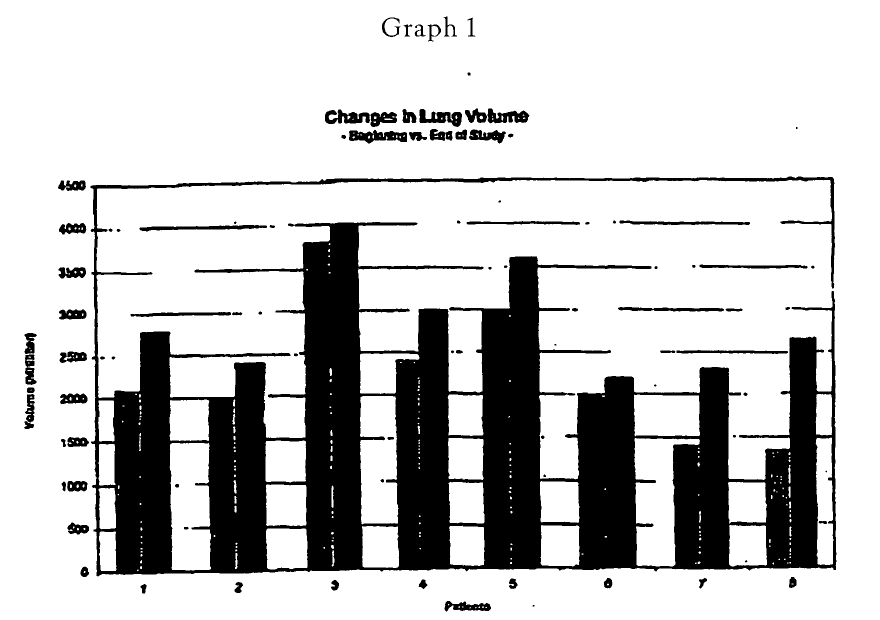

The concept of this invention was tested in a prospective clinical study with 18 tinnitus patients for a period of four months. The hearing capacity was objectively documented by audiometry. The before/after results of the audiometry measurements are shown in graph 2 hereafter;

Five patients had little or now improvement of their hearing capacity (0 - 10 decibel [dB]), eight patients had improvements between 10 and 20 dB, two patients between 20 and 30 dB, two patients between 30 and 40 dB and for one patient an improvement of hearing capacity between 40 and 50 dB was documented.

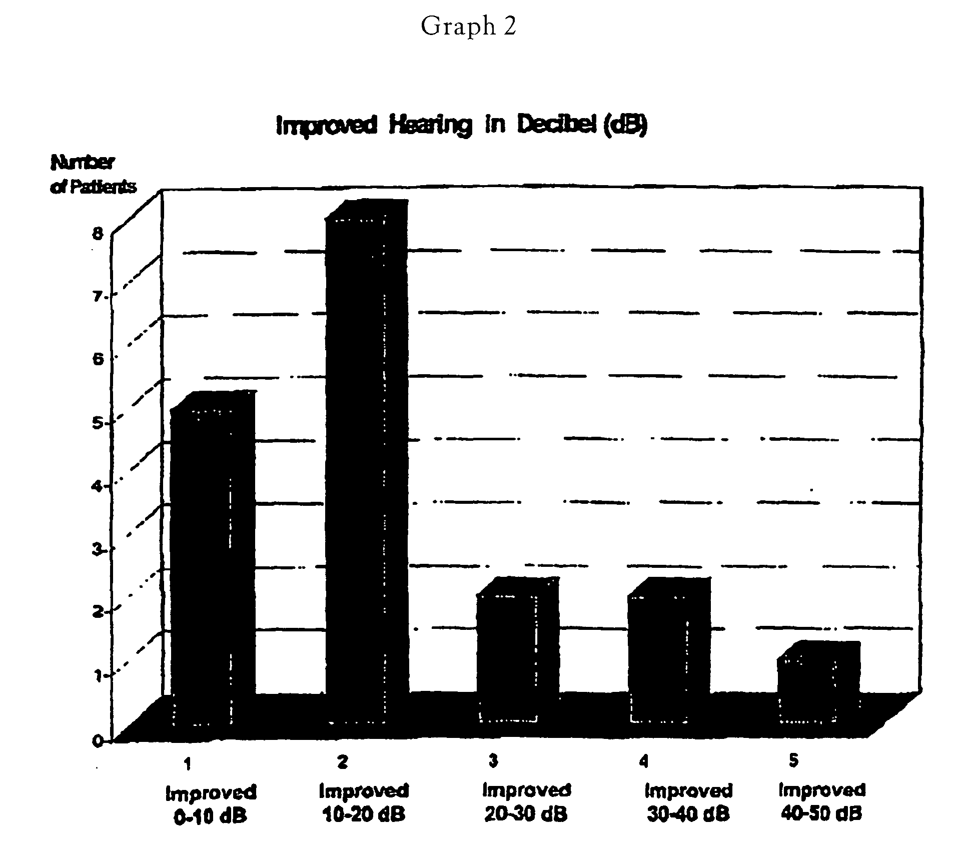

By now it is apparent that the administering of the compositions to patients being endangered of suffering or suffering from health conditions caused at least in part by constriction of smooth muscle cells in organs of their body.

## Claims

1. Use of a composition of biochemical substances consisting of
(i) at least one ascorbate compound selected from the group consisting of ascorbic acid, pharmaceutically acceptable ascorbate salts and mixtures thereof,
(ii) at least one arginine compound selected from the group consisting of arginine hydrochlorides, pharmaceutically acceptable arginine salts and mixtures thereof; and
(iii) at least one magnesium compound selected from the group consisting of magnesium, pharmaceutically acceptable magnesium salts and mixtures thereof
for the preparation of a pharmaceutical composition for treating tinnitus, asthma, glaucoma, infertility, spasms of the ureter and urethra, singultus, stomach cramps or cramps of the gall ducts.

2. Use of a composition of biochemical substances consisting of Ascorbic Acid, Ascorbyl Palmitate, Beta-, Gamma-, Delta-Tocopherol-Mix, Beta-Carotene, Biotin, Calcium Ascorbate, Calcium Citrate, Calcium Glycinate, Carotinoid-Mix: _{(A)pha-Carot., Lutein, Zea-, Kryptoxanthin)}, Cholecalciferol, Chromium Glycinate, Citrus Bioflavonoids, Coenzyme Q10, Copper Glycinate, Cyanocobalamin, d-Alpha-Tocopherol, d-Calcium Pantothenate, Dicalcium Phosphate, Folic Acid, Inositol, L-Arginine, L-Camitine, L-Cysteine, L-Lysine, L-Proline, L-Selenomethionine, Magnesium Ascorbate, Magnesium Citrate, Magnesium Glycinate, Manganese Chelate, Molybdenum Glycinate, Niacin, Niacinamid, Potassium Chelate, Pycnogenol, Pyridoxine, Riboflavin, Thiamine, and Zinc Glycinate for the preparation of a pharmaceutical composition for treating tinnitus, asthma, glaucoma, infertility, spasms of the ureter and urethra, singultus, stomach cramps or cramps of the gall ducts.

3. The use of claim 1, wherein the composition of biochemical substances is provided together with one or more biochemical substances selected from Ascorbic Acid, Ascorbyl Palmitate, Beta-, Gamma-, Delta-Tocopherol-Mix, Beta-Carotene, Biotin, Calcium Ascorbate, Calcium Citrate, Calcium Glycinate, Carotinoid-Mix: _{(Alpha-Carot., Lutein, Zea-, Kryptoxanthin)}, Cholecalciferol, Chromium Glycinate, Citrus Bioflavonoids, Coenzyme Q10, Copper Glycinate, Cyanocobalamin, d-Alpha-Tocopherol, d-Calcium Pantothenate, Dicalcium Phosphate, Folic Acid, Inositol, L-Arginine, L-Camitine, L-Cysteine, L-Lysine, L-Proline, L-Selenomethionine, Magnesium Ascorbate, Magnesium Citrate, Magnesium Glycinate, Manganese Chelate, Molybdenum Glycinate, Niacin, Niacinamid, Potassium Chelate, Pycnogenol, Pyridoxine, Riboflavin, Thiamine, and Zinc Glycinate.

4. The use of any one of claims 1 to 3, wherein said composition is to be administered to a patient in form of tablets, pills, injections, infusions, inhalations or suppositories.

5. Composition of biochemical substances consisting of Ascorbic Acid, Ascorbyl Palmitate, Beta-, Gamma-, Delta-Tocopherol-Mix, Beta-Carotene, Biotin, Calcium Ascorbate, Calcium Citrate, Calcium Glycinate, Carotinoid-Mix: _{(Alpba-Carot., Lutein, Zea-, Kryptoxanthin)}, Cholecalciferol, Chromium Glycinate, Citrus Bioflavonoids, Coenzyme Q10, Copper Glycinate, Cyanocobalamin, d-Alpha-Tocopherol, d-Calcium Pantothenate, Dicalcium Phosphate, Folic Acid, Inositol, L-Arginine, L-Carnitine, L-Cysteine, L-Lysine, L-Proline, L-Selenomethionine, Magnesium Ascorbate, Magnesium Citrate, Magnesium Glycinate, Manganese Chelate, Molybdenum Glycinate, Niacin, Niacinamid, Potassium Chelate, Pycnogenol, Pyridoxine, Riboflavin, Thiamine, and Zinc Glycinate.

6. The composition of claim 5, which is a pharmaceutical composition.

## Patentansprüche

1. Verwendung einer Zusammensetzung aus biochemischen Substanzen, die aus folgendem besteht:
(i) mindestens einer Ascorbat-Verbindung, die aus einer Gruppe ausgewählt ist, die aus Ascorbinsäure, pharmazeutisch akzeptablen Ascorbatsalzen und Mischungen davon besteht,
(ii) mindestens einer Argininverbindung, die aus einer Gruppe ausgewählt ist, die aus Argininhydrochlorid, pharmazeutisch akzeptablen Argininsalzen und Mischungen davon besteht, und
(iii) mindestens einer Magnesiumverbindung, die aus einer Gruppe ausgewählt ist, die aus Magnesium, pharmazeutisch akzeptablen Magnesiumsalzen und Mischungen davon besteht,
für die Herstellung einer pharmazeutischen Zusammensetzung zur Behandlung von Tinnitus, Asthma, Glaukom, Unfruchtbarkeit, Spasmen des Ureters und der Urethra, Schluckauf, Magenkrämpfen oder Krämpfen der Gallengänge.

2. Verwendung einer Zusammensetzung aus biochemischen Substanzen, die aus Ascorbinsäure, Ascorbylpalmitat, Beta-, Gamma-, Delta-Tocopherol-Gemisch, Beta-Carotin, Biotin, Kalziumascorbat, Kalziumcitrat, Kalziumglycinat, Carotinoid-Gemisch: _{(Alpha-Carot., Lutein, Zea-, Kryptoxanthin)}, Cholecalciferol, Chromglycinat, Zitrusbioflavonoide, Coenzym Q10, Kupferglycinat, Zyankobalamin, d-Alpha-Tocopherol, d-Kalzium-Pantothenat, Dikalziumphosphat, Folsäure, Inositol, L-Arginin, L-Carnitin, L-Cystein, L-Lysine, L-Prolin, L-Selenmethionin, Magnesiumascorbat, Magnesiumcitrat, Magnesiumglycinat, Manganchelat, Molybdenglycinat, Niazin, Niazinamid, Kaliumchelat, Pycnogenol, Pyridoxin, Riboflavin, Thiamin und Zinkglycinat besteht, für die Herstellung einer pharmazeutischen Zusammensetzung zur Behandlung von Tinnitus, Asthma, Glaukom, Unfruchtbarkeit, Spasmen des Ureter und der Urethra, Schluckauf, Magenkrämpfen oder Krämpfen der Gallengänge.

3. Verwendung nach Anspruch 1, wobei die Zusammensetzung aus biochemischen Substanzen zusammen mit einer oder mehreren biochemischen Substanzen, die aus Ascorbinsäure, Ascorbylpalmitat, Beta-, Gamma-, Delta-Tocopherol-Gemisch, Beta-Carotin, Biotin, Kalziumascorbat, Kalziumcitrat, Kalziumglycinat, Carotinoid-Gemisch: _{(Alpha-Carot., Lutein, Zea-, Kryptoxanthin)}, Cholecalciferol, Chromglycinat, Zitrusbioflavonoide, Coenzym Q10, Kupferglycinat, Zyankobalamin, d-Alpha-Tocopherol, d-Kalzium-Pantothenat, Dikalziumphosphat, Folsäure, Inositol, L-Arginin, L-Carnitin, L-Cystein, L-Lysine, L-Prolin, L-Selenmethionin, Magnesiumascorbat, Magnesiumcitrat, Magnesiumglycinat, Manganchelat, Molybdenglycinat, Niazin, Niazinamid, Kaliumchelat, Pycnogenol, Pyridoxin, Riboflavin, Thiamin und Zinkglycinat ausgewählt sind, bereitgestellt wird.

4. Verwendung nach einem der Ansprüche 1 bis 3, wobei die Zusammensetzung einem Patienten in Form von Tabletten, Pillen, Injektionen, Infusionen, Inhalationen oder Zäpfchen verabreicht werden soll.

5. Zusammensetzung aus biochemischen Substanzen, die aus Ascorbinsäure, Ascorbylpalmitat, Beta-, Gamma-, Delta-Tocopherol-Gemisch, Beta-Carotin, Biotin, Kalziumascorbat, Kalziumcitrat, Kalziumglycinat, Carotinoid-Gemisch: _{(Alpha-Carot., Lutein, Zea-, Kryptoxanthin)}, Cholecalciferol, Chromglycinat, Zitrusbioflavonoide, Coenzym Q10, Kupferglycinat, Zyankobalamin, d-Alpha-Tocopherol, d-Kalzium-Pantothenat, Dikalziumphosphat, Folsäure, Inositol, L-Arginin, L-Carnitin, L-Cystein, L-Lysine, L-Prolin, L-Selenmethionin, Magnesiumascorbat, Magnesiumcitrat, Magnesiumglycinat, Manganchelat, Molybdenglycinat, Niazin, Niazinamid, Kaliumchelat, Pycnogenol, Pyridoxin, Riboflavin, Thiamin und Zinkglycinat besteht.

6. Zusammensetzung nach Anspruch 5, die eine pharmazeutische Zusammensetzung ist.

## Revendications

1. Utilisation d'une composition de substances biochimiques, composée de :
a) au moins un composé d'ascorbate, choisi dans le groupe composé de l'acide ascorbique, de sels d'ascorbate acceptables d'un point de vue pharmaceutique et de mélanges de ceux-ci;
b) au moins un composé d'arginine, choisi dans le groupe composé de chlorhydrates d'arginine, de sels d'arginine acceptables d'un point de vue pharmaceutique et de mélanges de ceux-ci ; et
c) au moins un composé de magnésium, choisi dans le groupe composé du magnésium, de sels de magnésium acceptables d'un point de vue pharmaceutique et de mélanges de ceux-ci
pour la préparation d'une composition pharmaceutique destinée à traiter les acouphènes, l'asthme, le glaucome, l'infertilité, les spasmes de l'uretère et de l'urètre, le hoquet, les crampes d'estomac ou les crampes des voies biliaires.

2. Utilisation d'une composition de substances biochimiques, composée de : l'acide ascorbique ; le palmitate d'ascorbyle ; un mélange de bêta-, gamma-, delta-tocophérol ; le bêta-carotène ; la biotine ; l'ascorbate de calcium ; le citrate de calcium ; le glycinate de calcium ; un mélange caroténoïde : _{(alpha-carot., lutéine, zéa-, kryptoxanthine)} ; le cholécalciférol ; le glycinate de chrome ; des bioflavonoïdes d'agrumes ; le Coenzyme Q10 ; le glycinate de cuivre ; la cyanocobalamine ; le d-alpha-tocophérol ; le d-pantothénate calcique ; le phosphate dicalcique ; l'acide folique ; l'inositol ; la L-arginine ; la L-carnitine ; la L-cystéine ; la L-lysine ; la L-proline ; la L-sélénométhionine ; l'ascorbate de magnésium ; le citrate de magnésium ; le glycinate de magnésium ; un chélate de manganèse ; le glycinate de molybdène ; la niacine ; le niacinamide ; un chélate de potassium ; le pycnogénol ; la pyridoxine ; la riboflavine ; la thiamine ; et le glycinate de zinc, pour la préparation d'une composition pharmaceutique destinée à traiter les acouphènes, l'asthme, le glaucome, l'infertilité, les spasmes de l'uretère et de l'urètre, le hoquet, les crampes d'estomac ou les crampes des voies biliaires.

3. Utilisation selon la revendication 1, dans laquelle la composition de substances biochimiques est fournie avec une ou plusieurs substances biochimiques choisies parmi les : acide ascorbique ; palmitate d'ascorbyle ; mélange de bêta-, gamma-, delta-tocophérol ; bêta-carotène ; biotine ; ascorbate de calcium ; citrate de calcium ; glycinate de calcium; mélange caroténoïde : _{(alpha-carot., lutéine, zéa-, hyptoxanthine)} ; cholécalciférol ; glycinate de chrome ; bioflavonoïdes d'agrumes ; Coenzyme Q10 ; glycinate de cuivre ; cyanocobalamine ; d-alpha-tocophérol; d-pantothénate calcique ; phosphate dicalcique ; acide folique ; inositol ; L-arginine ; L-carnitine ; L-cystéine ; L-lysine ; L-proline ; L-sélénométhionine ; ascorbate de magnésium ; citrate de magnésium ; glycinate de magnésium ; chélate de manganèse ; glycinate de molybdène ; niacine; niacinamide; chélate de potassium ; pycnogénol ; pyridoxine ; riboflavine ; thiamine ; et glycinate de zinc.

4. Utilisation selon l'une quelconque des revendications 1 à 3, dans laquelle ladite composition est à administrer à un patient sous forme de comprimés, de pilules, d'injections, de perfusions, d'inhalations ou de suppositoires.

5. Composition de substances biochimiques composée de : l'acide ascorbique ; le palmitate d'ascorbyle ; un mélange de bêta-, gamma-, delta-tocophérol ; le bêta-carotène ; la biotine ; l'ascorbate de calcium ; le citrate de calcium ; le glycinate de calcium ; un mélange caroténoïde : _{(alpha-carot., lutéine, zéa-, kryptoxanthine)} ; le cholécalciférol ; le glycinate de chrome ; des bioflavonoïdes d'agrumes ; le Coenzyme Q10 ; le glycinate de cuivre ; la cyanocobalamine ; le d-alpha-tocophérol ; le d-pantothénate calcique ; le phosphate dicalcique ; l'acide folique ; l'inositol ; la L-arginine ; la L-carnitine ; la L-cystéine ; la L-lysine ; la L-proline ; la L-sélénométhionine ; l'ascorbate de magnésium ; le citrate de magnésium ; le glycinate de magnésium ; un chélate de manganèse ; le glycinate de molybdène ; la niacine ; le niacinamide ; un chélate de potassium ; le pycnogénol ; la pyridoxine ; la riboflavine ; la thiamine ; et le glycinate de zinc.

6. Composition, selon la revendication 5, qui est une composition pharmaceutique.
